# EUROPEAN PATENT APPLICATION

(11) **EP 3 666 314 A1**
(43) Date of publication of application: **17.06.2020**
(21) Application number: 18211425.6
(22) Date of filing: 10.12.2018
(51) Int. Cl.: A61M 5/20, A61M 5/32, A61M 5/145, A61M 5/28

(54) **INJECTION DEVICE**

(71) Applicant: Ares Trading S.A., 1170 Aubonne (CH)
(72) Inventor: LE MASNE, Quentin, 01220 Divonne les Bains (FR); LEWIS, Scott Alexander, Cambridge, CB4 0DW (GB); O'SHEA, Macdara Eamonn, Cambridge, CB4 0DW (GB); SHAW, Thomas Henry, Cambridge, CB4 0DW (GB); BYATT, Douglas Edward Cambbell, Cambridge, CB4 0DW (GB)
(74) Representative: Merck Serono S.A. Intellectual Property

(57) **Abstract**

Injection device (2) comprising a housing (3), a syringe cradle (12) for removably receiving a syringe (1) having a needle (7), a container (10) with a flange (11), a plunger (9) and a removable needle cap (8) in the housing, and a system for performing automated injection comprising:
- a cap actuation mechanism (4) for gripping, storing and retrieving the needle cap,
- a syringe actuation mechanism (5) for moving the syringe in an injection direction (*Id*) from a position where the needle is inside the housing to an injection position where the needle projects out of a needle port of the housing on a skin contact face of the housing, and
- a plunger actuation mechanism (6) for advancing the plunger for administration of the liquid drug.

## Description

### Field of the invention

The present invention relates to an automated injection device.

### Background

Many drugs are provided in syringes (pre-filled syringes) that are manually actuated, the syringe comprising a container with a needle and a plunger rod to expel the drug. The advantages of such standard pre-filled syringes are their widespread availability and the large range of medications that may be conditioned in such syringes. However, manual actuation of a syringe is often uncomfortable or difficult for certain patients and there are also safety risks associated with the needle cap not being put back properly over the needle after use of the syringe.

In order to provide an alternative and improve the ease of administration of a liquid drug to a patient automated or auto-injection devices have become very popular. Automated injection devices often are more convenient to operate by non-trained individuals such as for example a patient. However, such automated injection devices typically include a custom medicine container that is specially adapted for the injection device. Such medicine container may be a drug containing cartridge or a syringe (for example a pre-filled syringe). Various other injection devices may receive a standard size medicine container, for example a pre-filled syringe whereby the injection device is provided with the needle and needle actuation mechanism. Such systems however have similar drawbacks as the custom medicine container devices as the injection device only accepts the particular syringe. Both such specific syringes as well as custom medicine containers are often limited to a specific manufacturer or a specific medication, and the device may not be used with the delivery of medication from different manufacturers. Further, automated injection devices that receive cartridges require a needle that may only be used once and therefore needs to be provided in a disposable part, which often requires various steps to operate the device. In addition, ensuring the sterility prior to use may also be an issue when using such injection devices. Although automated injection devices are convenient for self-injection by a patient all of the aforementioned drawbacks result in high costs and increased risk of user error.

There remains a need for auto-injection devices which are easy to use and limit the number of steps needed to operate the device. At the same time, such improved auto-injection device would reduce needle stick injury from handling the medicine container, whether it is a cartridge and needle or a pre-filled syringe for example. There further exists a need for such auto-injection device to allow for a cost-effective administration of the medicine considering the number of disposable parts in administering medicine through injection.

### Summary of the invention

In view of the foregoing it would be an advantage to provide an automated injection device that is simple and safe to use and that does not rely on custom medicine container designs or is limited to a specific type of medicine container, whether this is a cartridge or a pre-filled syringe.

It is advantageous to provide an injection device that is reliable and easy to use and in particular that reduces the risk of false manipulation of the medicine container, such as a pre-filled syringe.

An object of this invention has been achieved by providing the automated injection device according to claim 1.

Another object of this invention has been achieved by providing the automated injection device according to claim 16.

Disclosed herein is an injection device comprising a housing, a syringe cradle for removably receiving a prefilled syringe having a needle, a barrel (possibly a glass barrel) with a flange, a plunger and a removable needle cap in the housing, and a system for performing automated injection comprising:
- a cap actuation mechanism for gripping, storing and retrieving the needle cap, whereby advantageously removing from and replacing the needle cap onto the syringe is with the aid of the syringe actuation mechanism,
- a syringe actuation mechanism for moving the syringe in an injection direction from a position where the needle is inside the housing to an injection position where the needle projects out of a needle port of the housing on a skin contact face of the housing and for moving the syringe in the opposite direction, and
- a plunger actuation mechanism for advancing the plunger for administration of the liquid drug.

According to a first aspect of the invention, the cap actuation mechanism comprises a movable gripper for holding and releasing the needle cap and an actuator to actuate the gripping and release function and to translate the gripper and therewith the needle cap from a position aligned with the needle to a position away from the injection path of the syringe.

According to a second aspect of the invention, the plunger actuation mechanism comprises an actuator paddle coupled to an output of an actuator motor configured to displace the syringe plunger, whereby in its fully rearward position the actuator paddle engages the syringe flange clamp to pull it backwards against the force of a spring such that the syringe flange may be easily inserted in a slot of the syringe cradle with some play.

In an advantageous embodiment, the actuator of the cap actuation mechanism comprises a motor and a screw-nut linear displacement mechanism having a screw driven by the motor and engaging a nut on the gripper, the gripper being mounted on slides fixed to a support frame.

In an advantageous embodiment, the gripper comprises a slidable separator finger and gripper jaws comprising a first jaw and a second jaw that are movably mounted with respect to each other, preferably on a platform which interfaces to the gripper jaws. The first jaw further comprises a first separator finger, whereby in an ungripped position the first separator finger and the slidable separator finger abut against each other to keep the first and second jaws in a defined open position one relative to the other, and whereby the slidable separator finger is movable to disengage the separator fingers and allow the first and second jaws to move to a gripped position.

In an advantageous embodiment, the slidable separator finger is laterally slidable in a transverse direction, Td, to the injection direction to disengage the separator fingers and allow the first and second jaws to move to a gripped position.

In an advantageous embodiment, the slidable separator finger is laterally slidable between lateral shoulders on the gripper, such that a lateral shoulder of the slidable separator finger abuts against a lateral shoulder of the gripper to translate the gripper in the transverse direction when the screw-nut is actuated.

In an advantageous embodiment, the slidable separator finger comprises a nut portion that is threadably engaged by the screw of said actuator of the cap actuation mechanism.

In an advantageous embodiment, the gripper comprises a spring mounted such as to biasing the first and second jaws together.

In an advantageous embodiment, the second jaw comprises a locking shoulder that engages an end edge of the needle cap such that the needle cap is clamped or gripped between the locking shoulder and a front wall on the first jaw.

In an advantageous embodiment, the syringe actuation mechanism comprises an actuator fixed in the housing and a movable syringe support frame including a syringe cradle for lodging the syringe coupled to an output of the actuator to displace the syringe support frame relative to the housing in the injection direction.

In an advantageous embodiment, the actuator comprises a motor and a screw-nut linear displacement mechanism having a screw driven by the motor and engaging a nut connected to the syringe support frame.

In an advantageous embodiment, the syringe cradle comprises a slot to receive the syringe flange with some play between a shoulder formed on the support frame and a clamp shoulder formed on a syringe flange clamp that is movably mounted relative to the syringe support frame and the housing to clamp the syringe flange against the shoulder.

In an advantageous embodiment, the syringe flange clamp is biased by a spring towards the clamping position.

In an advantageous embodiment, the plunger actuation mechanism comprises an actuator that is fixedly mounted to the syringe support frame such that the actuator displaces in the injection direction with the movement of the syringe support frame and couples to the syringe plunger to advance the plunger in the container during administration of the liquid drug.

In an advantageous embodiment, the actuator of the plunger actuation mechanism comprises a motor and a screw-nut mechanism to accomplish the corresponding linear movements of the syringe flange clamp and of the plunger for the drug administration operation.

In an advantageous embodiment, the syringe cradle is provided with a shape that allows insertion of the syringe into a base of the housing, the shape of the cradle being configured to conform to the shape of the syringe with a certain amount of play in order to allow easy insertion of the syringe in the cradle in the correct orientation, whereby the syringe may be held within the syringe cradle by flexible clip arms. It is to be understood that the correct orientation is that orientation after insertion of the syringe into the device in which the needle and needle cap of the syringe will occupy the gripping mechanism. It is further noted that the device allows for any rotational differences along the longitudinal axis of the syringe when placing the syringe into the device resulting in the operator not having to align the flanges of the syringe a fixed position.

Further objects and advantageous features of the invention will be apparent from the claims, from the detailed description, and annexed drawings.

### Brief description of the drawings

Figure 1a is a perspective view of an automated injection device according to an embodiment of the invention;
Figure 1b is a view of the device of figure 1a with a lid open and a syringe inserted in the device;
Figure 1c is a view similar to figure 1b with a portion of housing removed in order to see internal components of the device;
Figure 2 is a top view of the internal mechanism of the device illustrated in figure 1c wherein a needle cap removed from a syringe and the syringe is being actuated for injection;
Figure 3 is a perspective view of an actuator of a syringe actuation mechanism or of a plunger actuation mechanism of the injection device according to an embodiment of the invention;
Figures 4a and 4b are top and bottom views respectively of an internal mechanism of the injection device according to an embodiment of the invention in an initial position with a syringe that has just been loaded into the injection device;
Figure 5 is a view similar to figure 4a in a subsequent step with the syringe being clamped in the device;
Figures 6a and 6b illustrate the mechanism in a subsequent step;
Figures 7a to 7c illustrate perspective and plan views of a syringe cap gripper mechanism according to embodiment of the invention in an open state, and figures 8a to 8c show the gripper mechanism in a gripping state;
Figures 9a and 9b show the mechanism of figures 6a and 6b respectively in a subsequent step, the cap removed from the syringe body;
Figures 10a and 10b illustrate the cap being moved laterally in a subsequent step;
Figure 11 shows a subsequent needle insertion step,
Figure 12 shows a subsequent drug injection step;
Figure 13 shows a subsequent step illustrating retraction of the syringe;
Figure 14 shows a subsequent step in which the cap is moved to align with the syringe body;
Figure 15 shows a subsequent step it with the cap put back on to the syringe body; and Figures 16a to 16c show a subsequent step in which the cap is released by the gripper to allow removal of the syringe from the automated injection device.

### Detailed description of embodiments of the invention

Referring to the figures, an injection device 2 according to an embodiment of the invention comprises a housing 3 having a base portion 3a and lid portion 3b. The base 3a comprises a syringe cradle 12 for lodging therein a syringe 1. The syringe 1 may be a standard type of syringe that is in widespread use for manual transcutaneous injection of a liquid drug. Such transcutaneous injection may include for example subcutaneous injections. The syringe 1 comprises a needle 7, a barrel 10, possibly a glass barrel, having a flange 11, a plunger 9 for pressing the liquid out through the needle, and a needle cap 8 for covering the needle when the syringe is not in use.

The syringe cradle 12 is provided with a shape that allows insertion of the syringe 1 into the base 3a, the shape of the cradle being configured to conform to the shape of the syringe with a certain amount of play in order to allow easy insertion of the syringe in the cradle in the correct orientation. In particular, given the shape, dimensions and position of the container flange 11, plunger rod and cap, there is only one orientation which the syringe 1 may be in position within the base 3a. The syringe may be held within the syringe cradle 12 by flexible clip arms 40 where the lid 3b is in an opened position to keep the syringe from falling out of the cradle before the lid is closed. A syringe ejection mechanism 47, for instance comprising one or more pivoting lever arms that engage under the syringe container 10 configured to lift the syringe out of the cradle 12 with sufficient force to overcome the flexible clip holder 40 is provided, the ejection mechanism being activated by pressing a button 27. Once a user has positioned a prefilled syringe 1 in the syringe cradle 12 and closed the lid 3b over the base 3a, a closing switch 28 which in the illustrated embodiment is in the form of a tab connected to the base. This switch 28 which may be in the form of a tab which wraps partly around the lid may provide a signal to the electronic circuit of the injection device that the lid is closed to the base. This function may be performed by many different types of sensors, for instance a proximity sensor based on magnetic, capacitive or optical sensing technology for instance or a combination thereof. An electronic circuit (not shown) within the base and / or within the lid is provided to control operation of the injection device, the injection device further comprising a power source in a form of a battery (not shown). A user interface, comprising for instance a screen, for instance provided on the inside and / or outside of the lid 3b, as well as various status indicator lights and audio output, are provided in the injection device. The user interface is configured for entering commands and providing status and information to the user, for instance to indicate the actions to be undertaken and the actions that have been completed. In addition, the device may include a docking port 46, such as for example an USB port for connecting the device either to a power supply for example to recharge or to transmit data.

The injection device 2 comprises an internal mechanism for performing automated injection of the liquid drug, which includes: removing the needle cap 8, advancing the syringe 1 such that the needle 7 extends through a needle port 26 at a skin contact face 38, advancing the plunger rod 9 by an amount corresponding to the desired injection dose, followed by withdrawing the syringe 1 and the plunger rod, such that the needle 7 enters back into the base 3b, and recapping the needle 7. The lid can then be opened and the used syringe ejected out of the cradle 12 by pressing the button 27 to actuate the ejection lever, allowing a new prefilled syringe 1 to be inserted in the syringe cradle 12 for a subsequent administration.

The internal mechanism of the injection device generally includes, for performing the aforementioned operations: a cap actuation mechanism 4 for gripping, storing and retrieving the needle cap 8, whereby advantageously removing from and replacing the needle cap 8 onto the syringe is with the aid of the syringe actuation mechanism, a syringe actuation mechanism 5 for advancing and withdrawing the syringe 1 and therewith the needle 7, and a plunger actuation mechanism 6 for advancing the plunger 9 for administration of the liquid drug.

The cap actuation mechanism comprises a gripper 13 for holding the needle cap 8 and an actuator 14a to actuate the gripping function and to translate the needle cap 8 from a position aligned with the needle 7 to a position laterally disengaged from the syringe, and its return to a position aligned with the needle after administration of the liquid drug and retraction of the syringe. Further, locking of the gripper 13 in the release/open position is also achieved by the actuator 14a.

The syringe actuation mechanism 5 comprises an actuator 14b fixedly mounted relative to a support frame 16b that is static with respect to the housing base 3a. A support frame 16c that lodges the syringe 1 is coupled to an output of the actuator 14b such that the actuator 14b of the syringe actuation mechanism 5 can displace the support frame 16c and therewith the syringe 1 relative to the housing base 3a in an injection direction *Id.*

The plunger actuation mechanism 6 comprises an actuator 14c that is fixedly mounted to the syringe support frame 16c such that the actuator 14c displaces in the injection direction with the movement of the syringe support frame 16c. The actuator 14c of the plunger actuation mechanism couples to the plunger 9 to advance the plunger in the container during administration of the liquid drug.

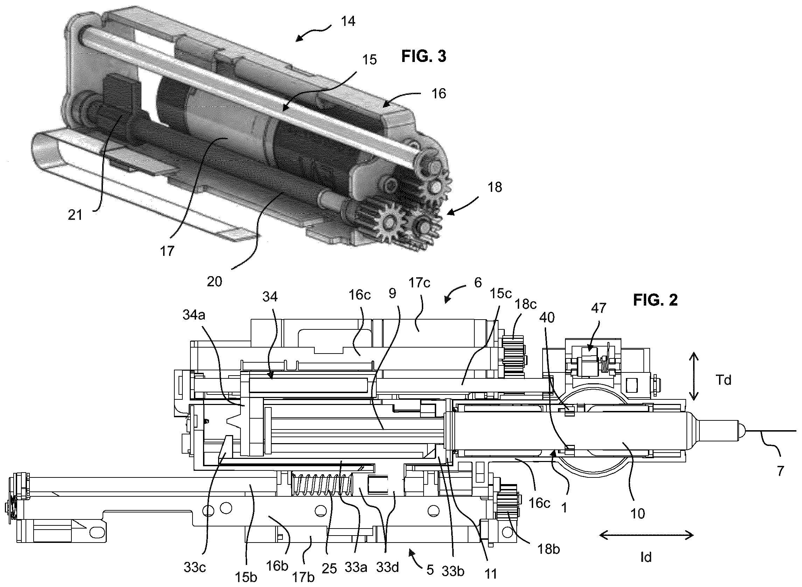

The actuators 14b, 14c of the syringe actuation mechanism 5 and plunger actuation mechanism 6 respectively, may have a construction as illustrated in figure 3, the actuator being generally indicated by reference number 14. The actuator 14 comprises an electrical motor 17 a screw-nut mechanism comprising a screw 20 and a nut 21. The screw 20 is coupled to the output rotor of the motor via a gear set 18, optionally a reduction gear set 18. A slide (i.e. a guide rail) 15 is also provided to slidably guide the support frame that is coupled to the nut 21. The screw 20, motor 17 and slide 15 are mounted to a support frame 16, it however being understood that the support frame may have various configurations and assembled from one or more parts for mounting not only the actuator but also housing parts in other structural elements of the device. Rotation of the screw 20 in a first direction, respectively in a second opposite direction, translates the nut 21, in a forward, respectively reverse movement. Nut 21 is shaped such as to provide an anti-rotational mechanism preventing its rotation but enabling its translation by the rotation of the screw 20. The actuators 14b, 14c of the plunger actuation mechanism 6 and syringe actuation mechanism 5 can be substantially identical or may have different dimensions and configurations to accomplish the corresponding linear movements of the syringe flange clamp 33, syringe support frame 16c, and of the plunger 9 for the needle insertion and retraction operations and for the drug administration operation. In the following description, the corresponding functional elements of the actuators 14b and 14c of the syringe actuation mechanism 5 and plunger actuation mechanism 6 respectively are designated with the same reference numerals as in relation to figure 3 but with the designation letter b or c depending on the actuator being referred to.

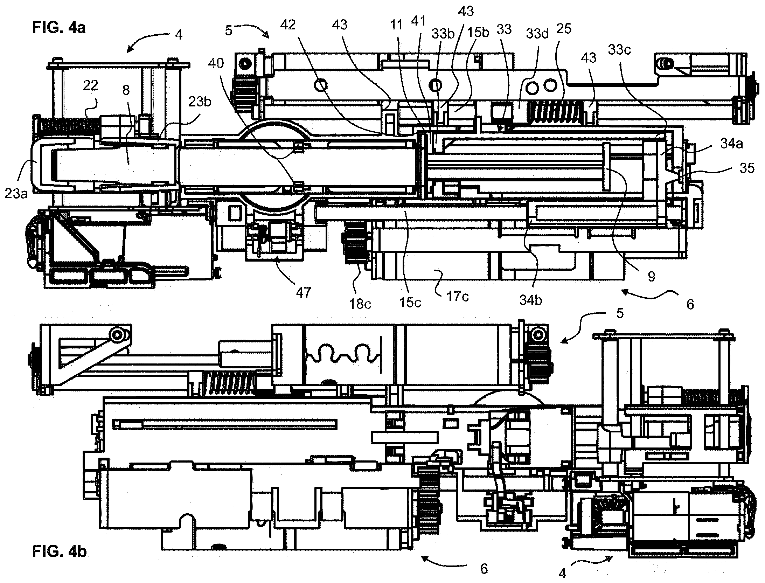

Referring now to figures 2 and figure 4, an initial step in the operation of the injection device 2 shall be described. In the configuration of figure 4, a syringe 1 has been inserted into the syringe cradle 12 and clipped elastically therein by elastic clip arms 40 so that it does not fall out of the cradle. The syringe flange 11 is received with some play within a slot 41 between a shoulder 42 formed on the syringe support frame 16c and a clamp shoulder 33b formed on a syringe flange clamp 33 that is movably mounted in the syringe support frame 16c.

The syringe flange frame 33 extends from the clamp shoulder 33b rearwardly to a distal end release shoulder 33c (best seen in figure 2) that is engaged by a shoulder (not shown) formed on an actuator paddle 34a of an actuator 34 of the plunger actuation mechanism 6. The syringe flange clamp 33 is mounted in the syringe support frame 16c but slidably guided and mounted on a guide 15b mounted on the support frame 16b fixed to the housing base 3a. The slide 15b also serves to slidingly guide the syringe support frame 16c. The syringe flange clamp 33 comprises a guide portion 33d that slidably inserts over the slide 15b and that is biased by a spring 25 engaging a guide portion 43 fixedly coupled or integrally formed with the fixed support frame 16b. The guide portion 43 thus provide the slidable guiding and holding of the syringe support frame 16c relative to the slide 15b which is fixedly mounted to the base 3a of the housing 3. The syringe support frame 16c can thus translate in the injection direction *Id,* guided by the slide 15b and advanced by the actuator 14b, whereby the nut 21 of the actuator (not shown in figure 4) is coupled to the syringe support frame 16c and advances or reverses depending on the direction of movement of the screw (which is not seen in figure 4 because it is below the slide 15b).

As shown in figure 4, the clamp shoulder 33b can make a small displacement in the injection direction *Id* relative to the syringe support frame 16c, whereby the spring 25 positioned therebetween biases the syringe flange clamp 33 in the forward direction towards the skin contact face 38. In the initial position allowing insertion of the syringe 1 in the syringe cradle 12, the actuator paddle 34a is in its fully rearward position and engages the distal end release shoulder 33c of the syringe flange clamp 33 to pull it backwards against the elastic force of the spring 25 such that the slot 41 allows the syringe flange 11 to be easily inserted in the slot 41 with some play.

Initial operation of the injection device comprises actuating the actuator 14c of the plunger actuator mechanism in order to advance by a small amount the actuator arm 34 coupled to the actuator paddle 34a such that the syringe flange clamp 33, under the force of the spring 25 advances until it abuts the flange 11 of the syringe and clamps it against the shoulder 42 of the syringe support flange 16c. The clamping of the syringe flange 11 thus holds and positions the syringe 1 in the cradle 12 in a defined position without any play.

The plunger actuation mechanism 6 comprises the actuator 14c with the components as described in relation to the actuator of figure 3 mounted to the syringe support frame 16c, the output of the motor 17c coupled to a plunger actuator arm 34. The actuator 14c of the plunger actuation mechanism thus moves in translation with the syringe in the injection direction *Id.* The plunger actuator arm 34 is slidably mounted via a guide portion 34b on a slide 15c extending in the injection direction *Id* and fixedly mounted to the syringe support frame 16c. A position sensor 35 may be provided on the syringe support frame 16c to detect the most rearward position of the plunger actuation arm 34.

The plunger actuator arm 34 comprises a plunger actuator paddle 34a that may serve two functions, a first being to retract and release the syringe flange clamp 33 against the spring force of the spring 25 as already described above, and a second being to press the plunger 9 of the syringe 1 during the injection operation.

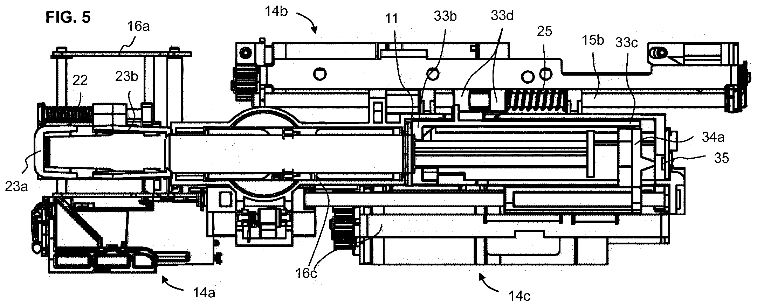

Referring now to figure 5, in a step subsequent to clamping of the syringe flange 11, the actuator 14b of the syringe actuation mechanism 5 is actuated to advance the syringe support frame 16c in the injection direction *Id* such that a portion of the gripper 13 of the cap actuation mechanism 4 is moved against the spring force of a spring 22 to reduce play between locking shoulder 29 of second gripper jaw 23b and the top of the needle cap in the gripper 13 within which the needle cap 8 is positioned. The gripper 13 of the cap actuation mechanism 4 comprises gripper jaws 23 comprising a first jaw 23a and a second jaw 23b that are movably mounted with respect to each other on a support frame (platform) such that they can be clamped to the needle cap 8 as it will be described further on.

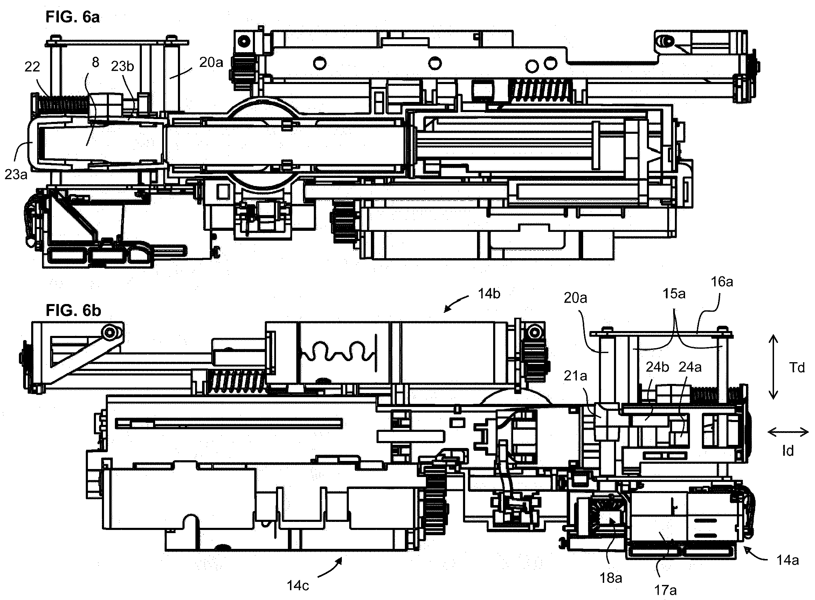

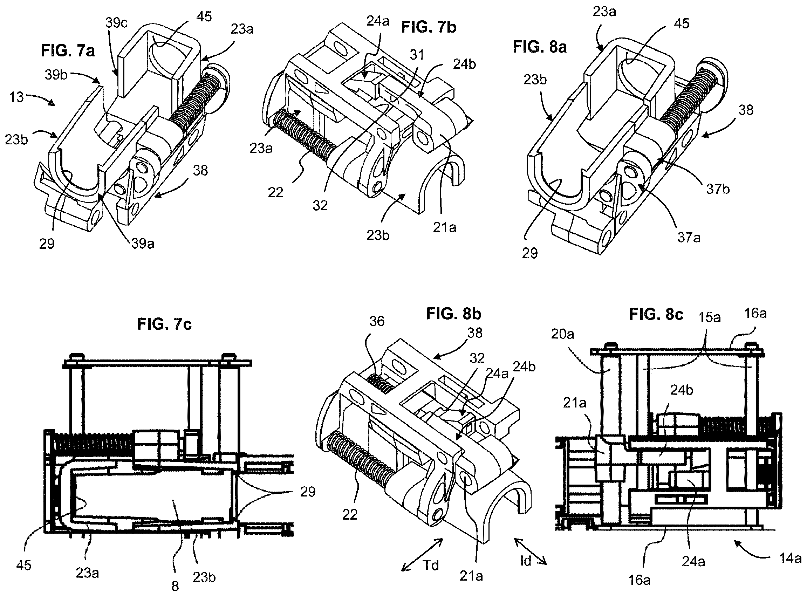

In a step subsequent to the aforementioned step, referring now to figures 6a to 8c, a gripping operation of the needle cap 8 will now be described. As best seen in figures 7a to 7c, in an initial ungripped position, the first and second gripper jaws 23a, 23b are kept in a defined position one relative to the other, whereby the first gripper jaw 23a is held in its defined position by separator fingers 24a, 24b that abut against each other, and the second gripper jaw 23b is held in its defined position being biased by spring 22. The separator finger 24a on one of the first gripper jaw 23a is fixedly formed or assembled to the gripper jaw 23a. The slideable separator finger 24b having a nut portion 21a is laterally slidable with respect to the gripper jaws 23a and 23b in a transverse direction *Td* between lateral shoulders 32 found on the gripper 13 such that the slideable separator finger 24b can move laterally to disengage the separator finger 24a as best seen in figure 8b. Once the separator fingers 24a, 24b disengage, a spring 36 mounted between the first gripper jaw 23a and the support frame, and biases first gripper jaw 23a towards the second gripper jaw 23b. As best seen in figures 8a and 6a, the jaw 23b comprises a locking shoulder 29 that engages an end edge of the needle cap 8 such that the needle cap is clamped or gripped between the locking shoulder 29 and a front wall 45 on the other jaw 23a.

The transverse displacement of the slideable separator finger 24b is performed by a screw 20a of a screw-nut mechanism of the actuator 14a of the cap actuation mechanism 4. The slideable separator finger 24b thus comprises a nut portion 21a that is threadably engaged by the screw 20a of the actuator 14a. Thus, for the cap gripping operation, the motor 17a of the actuator is actuated such that the slideable separator finger 24b (see figures 6a to 8c) performs a transverse displacement in the transverse direction *Td* sufficient to disengage the separator fingers 24a, 24b and allow the first gripper jaw 23a to bias towards the second gripper jaw 23b under the force of the spring 36, the motor being stopped in this disengaged position as illustrated in figures 8b and 8c.

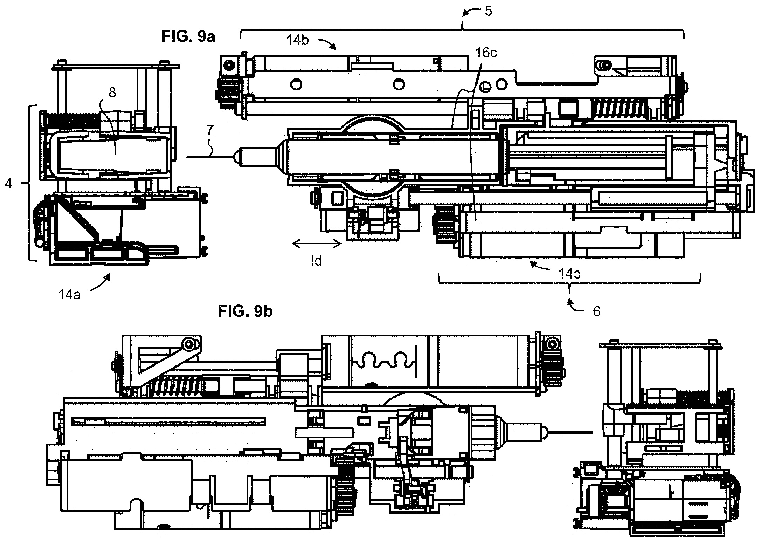

Referring to figures 9a and 9b, in a subsequent step, the actuator 14b of the syringe actuation mechanism 5 is actuated in a reverse direction to draw the syringe support frame 16c and the syringe 1 therewith rearwardly such that the needle 7 is disengaged completely from the needle cap 8.

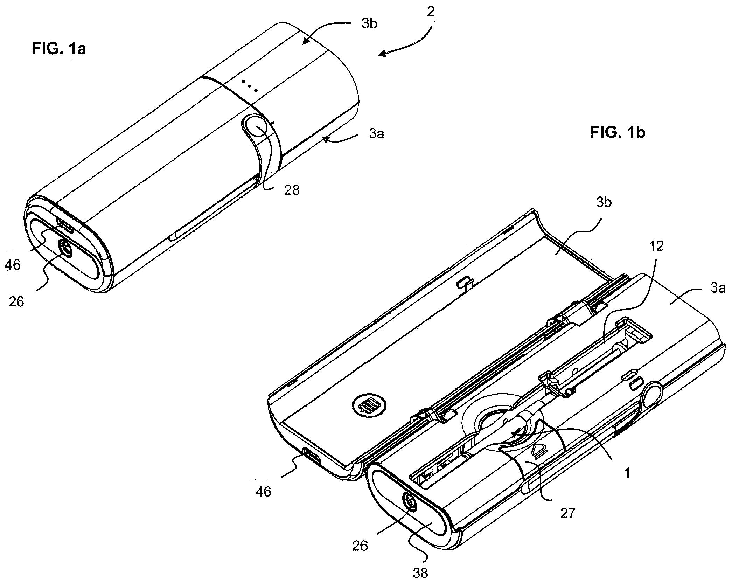

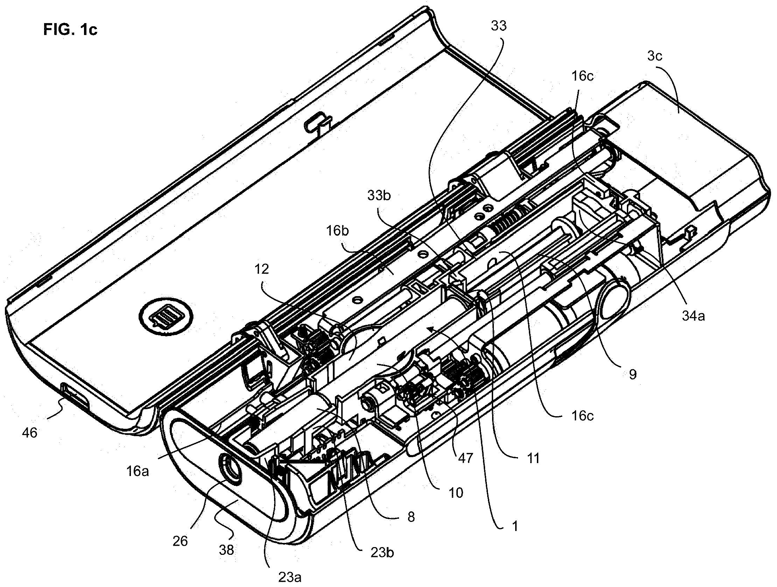

It may be noted in this position that the syringe support frame 16c extends rearwardly beyond the end of the housing 3 which may be provided with a moveable hat 3c to allow this rearward extension during the uncapping operation as is seen in Figure 1c. The rearwardly moveable hat 3c allows the housing of the injection device to have a more compact form and in particular a more compact length that is only extended during the uncapping operation for greater convenience to carry and store the device between uses.

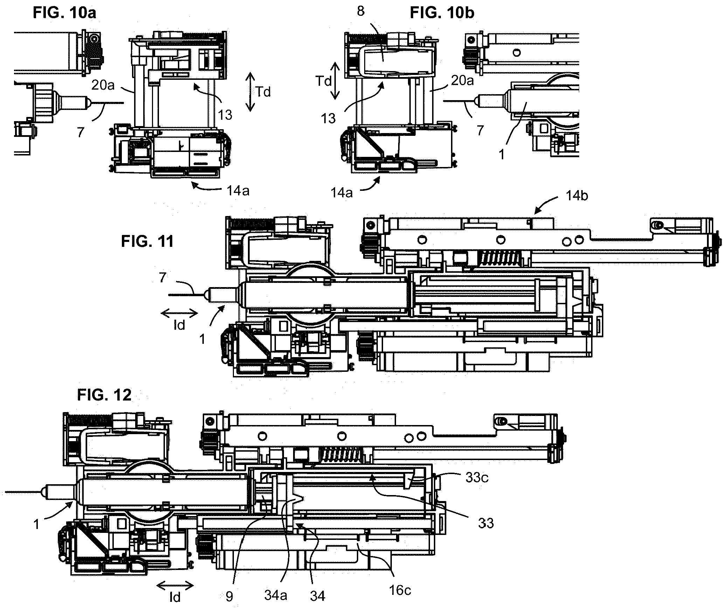

Referring to figures 10a and 10b, in a subsequent step, the gripper 13 holding the needle cap 8 is then fully translated by actuating the motor 17a that turns the screw 20a engaging the nut portion 21a on the slideable separator finger 24b. Since the slideable separator finger 24b abuts against a lateral shoulder 31 of the gripper 13, the gripper is translated in the transverse direction *Td* by sliding along the slides 15a on which the gripper 13 is slidably mounted and positioned. The slides 15a are fixed to a support frame 16a that holds the motor 17a and screw 20a of the actuator 14a fixedly to the base 3a of the housing. In the illustrated embodiment, the motor 17a is connected through bevelled gears 18a that allowed the motor to be positioned longitudinally in the injection direction Id and transmit a rotation to the screw 21a that extends in the transverse direction *Td.*

Referring now to figure 11, the actuator 14b of the syringe actuation mechanism 5 is actuated to advance the syringe support frame 16c and therewith the syringe 1 to a forward most position such that the needle 7 projects through the needle port 26. This occurs when the skin contact face 38 of the injection device is placed against the skin of a patient. It may be noted that a proximity sensor, that may for instance advantageously be placed on the lid 3a of the housing below or as part of the contact face 38, detects that the closed housing is positioned against the skin of the patient. Such proximity sensor may for instance detect a change in capacitance when in touch with the skin and thereby discriminate between a patient and an inanimate object. Various other physiological sensors may also be used to ensure that the injection device is activated only when it is in contact with the skin of a patient.

In a subsequent step as illustrated in figure 12, the drug may be injected by actuating the motor 17c of the plunger actuation mechanism 6 to advance the plunger actuation arm 34. The actuator paddle 34a presses against the distal end of the plunger 9 to push it into the syringe container 10.

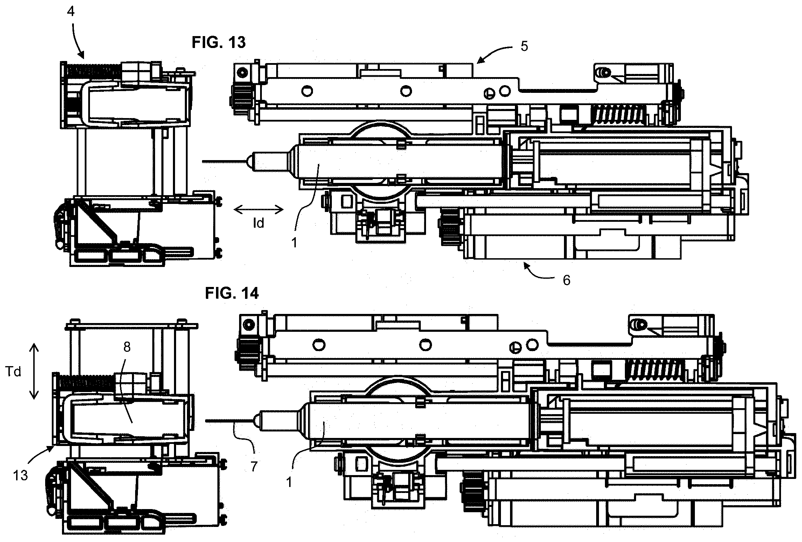

In the following step as illustrated in figure 13, the plunger actuator arm 34 is moved back to its rearward most position relative to the syringe support frame 16c and the syringe support frame 16c is moved in a reverse direction such that the needle is retracted into the housing 3 by the syringe actuation mechanism 5. The gripper 13 of the cap actuation mechanism is then translated back into its initial position by actuating the motor 17a to turn the screw 20a in a reverse direction. Since the slideable separator finger 24b is in the cap gripping position and abuts against the side of the other separator finger 24a, the gripper 13 which remains in a clamping position keeps holding the cap 8 until it translates fully back to the initial aligned position, aligned with the syringe 1 and needle 7.

In general, contact switches or proximity sensors may be positioned to detect the end of travel positions for each of the actuation mechanisms to stop the actuator motors once full displacement in a forward or rearward most position has occurred. The end of travel positions for each of the actuation mechanisms and to stop the respective actuator motors may also be sensed through the use of motor torque sensing through current monitoring or through sensing the number of revolutions of the actuator motor.

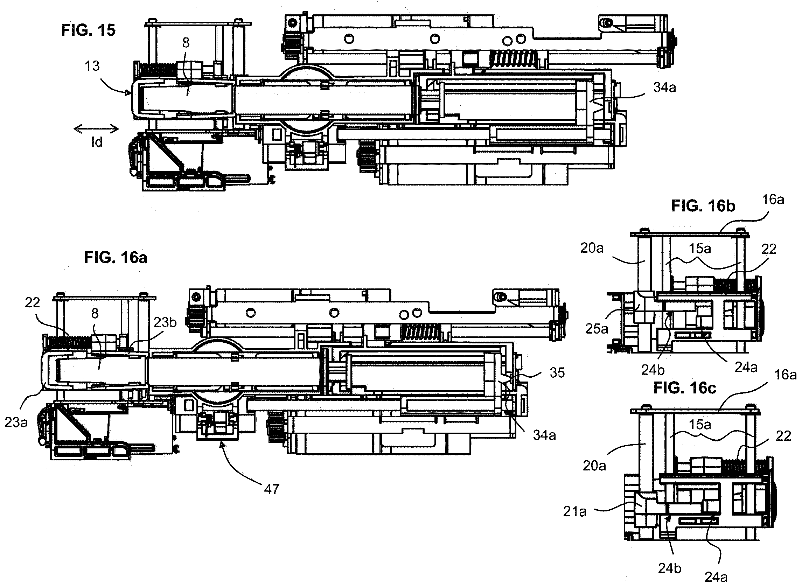

In a subsequent step, as illustrated in figure 15, the syringe actuation mechanism may be actuated to advance the syringe support frame 16c and therewith the syringe 1 until the needle 7 is capped.

In a subsequent step as illustrated in figure 16a to 16c, the syringe support frame 16c is further advanced such that end of the cap (8) pushes front wall 45 on jaw 23a moving the jaw 23a against the resistance of spring 36 such that the ends of the separator fingers 24a, 24b, as best seen in figure 16b, do not overlap and the cap actuation mechanism 4 can then be actuated to turn the screw 20a and displace the nut portion 21a of the slideable separator finger 24b such that the ends of the separator fingers 24a, 24b then align as shown in figure 16c. Subsequently, the actuator 14b of the syringe actuation mechanism 5 is actuated to reverse/withdraw the syringe support frame 16c in the injection direction Id such that the second gripper jaw 23b is biased towards the top of the gripper support frame 16a through the biasing spring force of spring 22 to reintroduce play between locking shoulder 29 of second gripper jaw 23b and the top of the needle cap 8. The gripper 13 of the cap actuation mechanism 4 is thus back to its initial position with play, and the actuator paddle 34 of the plunger actuation mechanism 6 is in its rearward most position to unclamp the syringe flange, to allow the used syringe to be removed from the injection device and a new syringe placed therein. The actuator paddle 34 of the plunger actuation mechanism 6 in its rearward most position may be detected by the sensor 35.

Advantageously, even in the absence of needle cap 8 in the gripper jaws, the gripper jaws can be returned from a locked/closed position into a release/open position. Thus, in the absence of a syringe 1 and needle cap 8 the injection device can recover and open the gripper jaws 23a, 23b. The second gripper jaw 23b being mounted on sliders on the gripper support frame 16a allow it to move in direction Id relative to the rest of the device. The second gripper jaw 23b has an endstop 37b which is biased by spring 22 towards endstop 37a of the platform 38, which platform 38 supports the gripper jaws 23a,23b. The second gripper jaw 23b further includes a first shoulder feature (39a) whereby the syringe support frame 16c can transmit movement in direction Id to the second gripper jaw 23b, against the bias of spring 22. Further, the first and second gripper jaws each have shoulder features (39b, 39c) whereby, in the absence of a cap these shoulder features abut against each other, allowing the transmission of movement in the injection direction Id from the second gripper jaw 23b to the first gripper jaw 23a.

When the separator fingers 24a and 24b are not aligned and the first gripper jaw 23a is biased towards the second gripper jaw 23b by spring 36 in the absence of a syringe 1 and needle cap 8, the further advancement of the syringe support frame 16c in the Id direction will translate the gripper 13 into the Id direction such that the ends of the separator fingers 24a and 24b do not overlap. The cap actuation mechanism 4 can then be actuated to turn the screw 20a and displace the nut portion 21a of the slideable separator finger 24b such that the ends of the separator fingers 24a, 24b then align. Thereby recovering and opening the gripper jaws 23a, 23b following actuation of the actuator 14b of the syringe actuation mechanism 5 to reverse/withdraw the syringe support frame 16c in the injection direction Id such that the second gripper jaw 23b is biased towards the top of the gripper support frame 16a (i.e. endstop 37b of the second gripper jaw 23b is biased against endstop 37a of platform 38) through the biasing spring force of spring 22 to open the gripper jaws 23a, 23b from each other. The gripper 13 of the cap actuation mechanism 4 is thus back to its initial position.

### List of references used

**Syringe** 1
   Needle 7
   Needle cap 8
   Plunger 9
   Container 10
      Flange 11
**Injection device** 2
   Housing **3**
      Base 3a
         Syringe cradle 12
            Elastic clip arms 40
            Slot 41 for syringe flange
      Lid 3b
      skin contact face 38
      Needle port 26
      Syringe ejection mechanism
         button 27
      Moveable hat 3c
   closing switch 28
   syringe ejection mechanism 47
   docketing port 46
   support frame 16b
   **Cap actuation mechanism 4**
      Gripper 13
         Spring 22
         gripper jaws 23
            lateral shoulders 32
            first jaw 23a
               first separator finger 24a,
               first jaw shoulder feature 39c
               front wall 45
            second jaw 23b
               locking shoulder 29
               second jaw endstop 37b
               second jaw shoulder features 39a, 39b
         platform 38
            slideable separator finger 24b
               guide 30
               lateral shoulder 31
               nut portion 21a
            spring 36
            platform endstop 37a
      Actuator 14a
         Motor 17a
            gears 18a
      Screw-nut mechanism
         Screw 20a
         Nut portion 21a
      Slide 15a
      Support frame 16a
**Syringe actuation mechanism 5**
   Actuator 14b
      Motor 17b
         Reduction gears 18b
      Screw-nut mechanism
         Screw 20b
         Nut 21 b
   Slide 15b
   Support frame 16c
      shoulder 42
   Syringe flange clamp 33
      Clamp arm 33a
      Clamp shoulder 33b
      Distal end release shoulder 33c
      Guide 33d
      Spring 25
      guide portion 43
**Plunger actuation mechanism 6**
   Actuator 14c
      Motor 17c
         Reduction gears 18c
      Screw-nut mechanism
         Screw 20c
         Nut 21c
      Actuator arm 34
         Actuator paddle 34a
         Guide 34b
   Slide 15c
   Support frame 16c
*Injection direction Id*
*Transverse direction Td*

## Claims

1. Injection device (2) comprising a housing (3), a syringe cradle (12) for removably receiving a syringe (1) having a needle (7), a container (10) with a flange (11), a plunger (9) and a removable needle cap (8) in the housing, and a system for performing automated injection comprising:
- a cap actuation mechanism (4) for gripping, storing and retrieving the needle cap,
- a syringe actuation mechanism (5) for moving the syringe in an injection direction (*Id*) from a position where the needle is inside the housing to an injection position where the needle projects out of a needle port of the housing on a skin contact face of the housing, and
- a plunger actuation mechanism (6) for advancing the plunger for administration of the liquid drug,
wherein the cap actuation mechanism comprises a movable gripper (13) for holding and releasing the needle cap and an actuator (14a) to actuate the gripping and release function and to move the gripper and therewith the needle cap from a position aligned with the needle to a position disengaged from the syringe.

2. Injection device according to claim 1 wherein the actuator (14a) comprises a motor (17a) and a screw-nut linear displacement mechanism having a screw driven by the motor and engaging a nut on the gripper, the gripper being mounted on slides (15a) fixed to a support frame (16a).

3. Injection device according to claim 1 or 2 wherein the gripper comprises gripper jaws comprising a first jaw (23a) and a second jaw (23b) that are movably mounted with respect to each other, the first jaw having a separator finger (24a) whereby in an ungripped position the separator finger (24a) abut against a slideable separator finger (24b) to keep the first and second jaws in a defined open position one relative to the other, and whereby the slideable separator finger (24b) is movable to disengage the separator fingers and allow the first and second jaws to move to a gripped position.

4. Injection device according to the preceding claim wherein the slideable separator finger (24b) is laterally slidable in a transverse direction (*Td*) to the injection direction (*Id*) to disengage the separator fingers and allow the first and second jaws to move to a gripped position.

5. Injection device according to either of the two directly preceding claims wherein the slideable separator finger (24b) is laterally slidable between lateral shoulders (32) on the gripper, such that a lateral shoulder (31) of the slideable separator finger abuts against a lateral shoulder (32) of the gripper (13) to translate the gripper in the transverse direction (Td) when the second separator is actuated.

6. Injection device according to any of the three directly preceding claims wherein the slideable separator finger (24b) comprises a nut portion (21a) that is threadably engaged by the screw (20a) of said actuator (14a) of the cap actuation mechanism.

7. Injection device according to any preceding claim wherein the gripper comprises a spring (36) biasing the first jaw (23a) towards the second jaw (23b).

8. Injection device according to any preceding claim wherein the second jaw (23b) comprises a locking shoulder (29) that engages an end edge of the needle cap (8) such that the needle cap is clamped or gripped between the locking shoulder and a front wall (45) on the first jaw (23a).

9. Injection device according to any of preceding claim wherein the syringe actuation mechanism (5) comprises an actuator (14b) fixed in the housing and a movable syringe support frame (16c) for lodging the syringe coupled to an output of the actuator (14b) to displace the syringe support frame relative to the housing in the injection direction (*Id*).

10. Injection device according to the preceding claim wherein the actuator (14b) comprises a motor (17b) and a screw-nut linear displacement mechanism having a screw driven by the motor and engaging a nut connected to the syringe support frame (16c).

11. Injection device according to any preceding claim wherein the syringe cradle comprises a slot (41) to receive the syringe flange (11) with some play between a shoulder (42) formed on the support frame (16b) and a clamp shoulder (33b) formed on a syringe flange clamp (33) that is movably mounted relative to the syringe support frame (16c) and the housing to clamp the syringe flange against the shoulder.

12. Injection device according to the preceding claim wherein the syringe flange clamp (33) is biased by a spring (25) towards the clamping position.

13. Injection device according to the preceding claim wherein the plunger actuation mechanism (6) comprises an actuator paddle (34a) coupled to an output of an actuator motor (17c) configured to displace the syringe plunger, whereby in its fully rearward position the actuator paddle engages the syringe flange clamp (33) to pull it backwards against the force of the spring (25) such that the slot (41) allows the syringe flange (11) to be easily inserted in the slot with some play.

14. Injection device according to any preceding claim wherein the plunger actuation mechanism (6) comprises an actuator (14c) that is fixedly mounted to the syringe support frame (16c) such that the actuator displaces in the injection direction with the movement of the syringe support frame (16c) and couples to the syringe plunger (9) to advance the plunger in the container during administration of the liquid drug.

15. Injection device according to any preceding claim wherein the actuator of the plunger actuation mechanism comprises a motor and a screw-nut mechanism to accomplish the corresponding linear movements of the syringe flange clamp (33) and of the plunger (9) for the drug administration operation.

16. Injection device (2) comprising a housing (3), a syringe cradle (12) for removably receiving a syringe (1) having a needle (7), a container (10) with a flange (11), a plunger (9) and a removable needle cap (8) in the housing, and a system for performing automated injection comprising:
- a cap actuation mechanism (4) for gripping, storing and retrieving the needle cap,
- a syringe actuation mechanism (5) for moving the syringe in an injection direction *(Id)* from a position where the needle is inside the housing to an injection position where the needle projects out of a needle port of the housing on a skin contact face of the housing, and
- a plunger actuation mechanism (6) for advancing the plunger for administration of the liquid drug,
wherein the syringe cradle comprises a slot (41) to receive the syringe flange (11) with some play between a shoulder (42) formed on the support frame (16b) and a clamp shoulder (33b) formed on a syringe flange clamp (33) that is movably mounted relative to a syringe support frame (16c) and the housing to clamp the syringe flange against the shoulder.

17. Injection device according to the preceding claim further comprising any one or more of the features of claims 1-10 and 12-15.
